(19) Europäisches Patentamt
European Patent Office
Office européen des brevets

(11) Publication number: 0 002 288
B1

(12) EUROPEAN PATENT SPECIFICATION

(45) Date of publication of patent specification: 12.05.82

(21) Application number: 78200195.2

(22) Date of filing: 18.09.78

(51) Int. Cl.³: C 07 C 121/75, C 07 C 121/66, C 07 C 149/415, A 01 N 37/34

# (54) Process for the preparation of alpha-cyanobenzyl esters.

(30) Priority: 28.09.77 GB 4033577

(43) Date of publication of application: 13.06.79 Bulletin 79/12

(45) Publication of the grant of the patent: 12.05.82 Bulletin 82/19

(84) Designated Contracting States: CH DE FR GB NL

(56) References cited:
DE - A - 2 231 312
DE - A - 2 547 534
DE - A - 2 635 327
DE - A - 2 708 590

(73) Proprietor: SHELL INTERNATIONALE RESEARCH MAATSCHAPPIJ B.V.
Carel van Bylandtlaan 30
NL-2596 HR DEN HAAG (NL)

(72) Inventor: Reinink, Arend
Badhuisweg 3
NL-1031 CM Amsterdam (NL)
Inventor: Sheldon, Roger Arthur
Badhuisweg 3
NL-1031 CM Amsterdam (NL)

(74) Representative: Rogers, Roy Charles et al,
4 York Road
London SE1 7NA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 002 288 B1

Courier Press, Leamington Spa, England.

Process for the preparation of alpha-cyanobenzyl esters

The invention relates to a process for the preparation of *alpha*-cyanobenzyl esters of carboxylic acids. Some of these esters, especially the *alpha*-cyano-3-phenoxybenzyl esters, give rise to the important pesticides known as pyrethroids.

It is known to prepare *alpha*-cyanobenzyl esters by the reaction of a benzaldehyde with an acid halide and an alkali metal cyanide.

According to Offenlegungsschrift 2,708,590 it is also known to prepare *alpha*-cyanobenzyl esters by the reaction of a benzaldehyde with an acid halide and an alkali metal cyanide in the presence of a phase-transfer catalyst. Generally, this process produces *alpha*-cyanobenzyl esters in good yield but from time to time, an unwanted acid anhydride by-product is produced. This acid anhydride is derived from the acid chloride reactant which, in the case of the pyrethroid esters, is a very costly material so that any procedure which would avoid the waste of this costly material would be of considerable benefit.

The Applicant has now found a simple procedure whereby the acid anhydride by-product is substantially completely converted into its *alpha*-cyanobenzyl ester.

Accordingly, the present invention provides a process for the preparation of an ester of the general formula:—

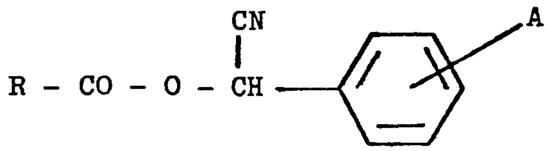

(I)

wherein R represents an optionally-substituted hydrocarbyl group and A represents a hydrogen atom or a phenoxy, phenylthio or benzyl group by reacting a benzaldehyde of the general formula:—

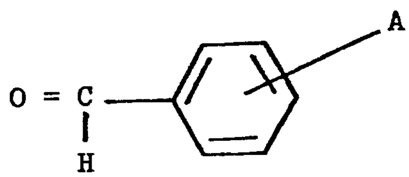

(II)

wherein A has the same meaning as in formula I with an acid halide of formula R—CO—Hal where Hal is chlorine or bromine and R has the same meaning as in formula I in the presence of water and a water-soluble cyanide and optionally in the presence of a phase-transfer catalyst characterised in that when the reaction slows or comes to a standstill a compound capable of generating cyanide ions is added to the reaction mixture so that by-product acid anhydride of the general formula:—

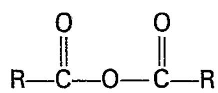

(III)

wherein R has the same meaning as in formula I is caused to react with the remaining aldehyde of formula II and with the generated cyanide ions to form further ester of general formula 1.

It has been found that when the invention slows or comes to a standstill the addition to the reaction mixture of a compound generating cyanide ions will restore the formation of the ester of general formula I, usually until the benzaldehyde present has been fully converted. A compound capable of generating cyanide ions is an alkaline compound, i.e. a compound capable of increasing the pH of the aqueous phase, for example a carbonate or hydroxide of an alkali metal such as sodium or potassium.

It is believed that the hydrogen halide liberated from acid anhydride formation produces so much free hydrogen cyanide from the water-soluble cyanide present that the reaction stops because of the lack of available cyanide ions. The addition of a compound capable of generating cyanide ions to the reaction mixture thus causes the reaction to re-start; when an alkaline compound is added to the reaction mixture its presence liberates cyanide ions from the hydrogen cyanide and by this means the reaction restarts.

The process according to the present invention is preferably carried out in the presence of a substantially water-immiscible aprotic solvent. A "substantially water-immiscible aprotic solvent" is defined as an aprotic solvent in which the solubility in water is not more than 5%v, at the reaction temperature adopted.

Examples of very suitable substantially water-immiscible aprotic solvents are alkanes or cycloalkanes, particular examples being *n*-heptane, *n*-octane, *n*-nonane, *n*-decane and their isomers (for

example, 2-methylpentane, 3-methylpentane, 2-methylhexane, 3-methylhexane and 2,4,4-trimethylpentane) and cyclohexane and methylcyclohexane.

Other very suitable substantially water-immiscible aprotic solvents are aromatic hydrocarbons and chlorinated hydrocarbons, for example benzene, toluene, *o*-, *m*-, and *p*-xylene, the trimethylbenzenes, dichloromethane, 1,2-dichloroethane, chloroform, monochlorobenzene and 1,2- and 1,3-dichlorobenzene. Very good results have been obtained with toluene.

Other examples of substantially water-immiscible aprotic solvents are dialkyl ethers and substantially water-immiscible alkanones, for example di-isopropyl ether and di-isobutyl ketone.

Mixtures of solvents, for example of alkanes, cycloalkanes and/or aromatic hydrocarbons may be employed, such as mixtures of *n*-heptane and toluene or of *n*-heptane and one or more xylenes. Gasolines rich in alkanes are very suitable, for example those with a boiling range at atmospheric pressure between 40 and 65°C or 80 and 110°C and particularly between 60 and 80°C. Mixtures of xylenes are also very suitable.

The esters of formula I may be prepared in the presence or absence of a phase-transfer catalyst. The phasetransfer catalyst may be any reagent which is capable of accelerating interphase reactions in aqueous/organic two-phase systems.

The phase-transfer catalyst may be an onium compound, particularly a quaternary onium compound of the general formula:

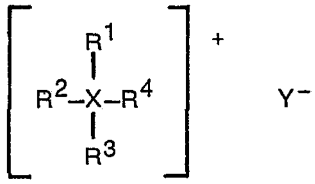

wherein X represents a nitrogen, phosphorus or arsenic atom, each of the groups $R^1$, $R^2$, $R^3$ and $R^4$ represent an alkyl, aralkyl, alkaryl or aryl group and Y represents a monovalent ion, e.g. a halide, such as chloride, bromide or iodide or an alkyl sulphate, such as methylsulphate or ethylsulphate or a sulphonium compound of the general formula:

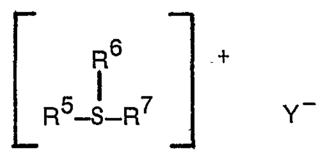

wherein each of the groups $R^5$, $R^6$ and $R^7$ represents an alkyl group and Y a monovalent ion, e.g., a halide, such as chloride, bromide or iodide, or an alkylsulphate, such as methylsulphate or ethylsulphate. Preferably, the alkyl groups contain 1 to 18 carbon atoms and the aralkyl and alkaryl groups contain up to 10 carbon atoms; the aryl group is preferably phenyl.

Examples of onium compounds are tetra-*n*-butylammonium bromide, tetra-*n*-butylammonium chloride, methyl-tri(2-methylphenyl)ammonium chloride, tetramethylphosphonium iodide, tetra-*n*-butylphosphonium bromide, methyltriphenylarsonium iodide, ethyl-2-methylpentadecyl-2-methyl-undecylsulphonium ethylsulphate, methyldinonylsulphonium methylsulphate and *n*-hexadecyl-dimethylsulphonium iodide. Very good results have been obtained with quaternary ammonium compounds.

The onium compound may be a hydroxide or a salt and can be employed as the functional portion of a strongly-basic anion-exchange resin having a structural portion (polymer matrix) and a functional portion (ion-active group). Of special importance are polystyrene resins, such as copolymers of aromatic monovinyl compounds and aromatic polyvinyl compounds, particularly styrene/divinylbenzene copolymers. The functional portion is a quaternary ammonium, phosphonium or arsonium group. Examples of strongly-basic anion exchange resins which may be employed are those derived from trimethylamine (such as the products known under the trade names of "Amberlite IRA—400," "Amberlite IRA—401," "Amberlite IRA—402," "Amberlite IRA—900," Duolite A—101—D," "Duolite ES—111," "Dowex 1," "Dowex 21K" and "Ionac A—450"), and those derived from dimethylethanolamine (such as the products known under the trade names of "Amberlite IRA—401," "Amberlite IRA—911," "Dowex 2," "Duolite A—102—D," "Ionac A—542," and "Ionac A—550").

Other phase-transfer catalysts are macrocyclic polyethers known as "crown ethers." These compounds, together with their preparations, are described in the literature, for example in Tetrahedron Letters No. 18 (1972), pages 1793—1796, and are commonly designated by referene to the total number of atoms forming the macrocyclic ring together with the number of oxygen atoms in that ring. Thus, the macrocyclic polyether whose formal chemical name is 1,4,7,10,13,16-hexaoxacyclo-

octadecane is designated "18-crown-6." Other examples of suitable macrocyclic polyethers are 3,4-benzo-1,6,9,12,15,18,21-heptaoxacyclotricos-3-ene and 3,4-benzo-1,6,9,12-tetraoxacyclotetradec-3-ene.

Other phase-transfer catalysts are surface-active agents. A "surface-active agent" is defined as in Kirk-Othmer, "Encyclopedia of Chemical Technology," second edition, volume 19 (1969), page 508: "An organic compound that encompasses in the same molecule two dissimilar structural groups, one being water soluble and one being water-insoluble."

The surface-active agent may be non-ionic, such as a poly(alkylene-oxy) derivative formed by reacting a higher alcohol, alkylphenol or fatty acid with ethylene oxide or propylene oxide. Suitable alcohols, alkylphenols or fatty acids contain an alkyl group of 8 to 20 carbon atoms; the number of alkylene-oxy units is in the range of 1 to 50. A suitable non-ionic surface-active agent (referred to by the trade name "Dobanol 91—6") is formed from a $C_9$—$C_{11}$-*n*-alkanol mixture and contains an average of six ethylene-oxy units. The non-ionic surface-active agent may be an alkylbenzene containing a straight-chain alkyl group. Suitable alkylbenzenes contain an alkyl group of 8 to 20 carbon atoms.

The molar ratio of the phase-transfer catalyst to the benzaldehyde of the general formula II may vary within wide limits, but is suitably from 1:5 to 1:10,000. Low molar ratios will require long reaction times, while high molar ratios will increase the cost involved in producing a given quantity of ester. Thus the choices of reaction time and molar ratio of catalyst to benzaldehyde are interdependent, and will in any individual instance be dictated by local economic factors. Very good results are usually obtained at molar ratios of from 1:10 to 1:500.

The molar ratio of water-soluble cyanide to benzaldehyde is suitable from 1.5:1 to 1.0:1.0 and preferably from 1.3:1 to 1.02:1.00. By "water-soluble cyanide" is meant a water-soluble salt of hydrogen cyanide. Of the water-soluble cyanides alkali-metal cyanides and alkaline-earth-metal cyanides are preferred. Sodium cyanide is particularly preferred, because it affords the ester of the general formula I in the shortest reaction time.

The process according to the present invention may be conducted starting from unsaturated or saturated aqueous solutions of water-soluble cyanide, and in the latter case in the presence or absence of solid water-soluble cyanide.

In the general formula I, A preferably represents a phenoxy group because this substituent gives rise to the most insecticidally-active from of the compound.

The group R in the general formula I is defined as an optionally-substituted hydrocarbyl group. This hydrocarbyl group may be, for example, an alkyl group, a cycloalkyl group or an aryl group. The alkyl group may be linear or branched and preferably contains up to 10 carbon atoms. The alkyl group may have a primary, secondary, tertiary or a quaternary carbon atom bound to the group —C(O)—, for example, methyl, isopropyl, 2,2-dimethylethyl and 2-methylpropyl. The alkyl group may carry substituents, such as hydrocarbyloxy or substituted phenyl groups, e.g. a halophenyl group, the preferred alkyl group being 1-(4-chlorophenyl)-2-methylpropyl. When R represents a cycloalkyl group, it preferably contains 3 to 6 carbon atoms and has as optional substituents one or more groups selected from alkyl, alkenyl or haloalkenyl, each of which suitably contains up to 8 carbon atoms. Examples of cycloalkyl groups are cyclopropyl, cyclobutyl and cyclohexyl groups.

The preferred meaning for R is an alkyl group optionally substituted by halophenyl or cyclopropyl substituted by one or more methyl or dihalovinyl groups. Very good results have been obtained in the preparation of esters based on optionally-substituted cyclopropanecarboxylic halides, particularly with 2-(2,2-dihalovinyl)-3,3-dimethylcyclopropanecarboxylic halide. The corresponding anhydride of the latter acid halide is novel and can be prepared in a manner known per se, for example by reacting 2-(2,2-dihalovinyl)-3,3-dimethylcyclopropanecarbonyl chloride with 2-(2,2-dihalovinyl)-3,3-dimethylcyclopropanecarboxylic acid. If applicable, the acid haldes, the anhydrides, and the esters may have a *cis-* or *trans*-structure or may be a mixture of such structures and may be a pure optical isomer or a mixture of optical isomers.

The temperature at which the process is conducted is suitably above 0°C and is preferably in the range of from 10°C to 50°C. Very good results have been obtained at temperatures in the range of from 15°C to 40°C. The process has the advantage that it is suitably carried out at ambient temperatures.

The process according to the invention may be carried out by gradual addition of the water-soluble cyanide to a mixture, preferably a stirred mixture, of the other starting compounds. Alternatively, all materials may be combined and the reaction allowed to take place with vigorous stirring of the reaction mixture.

The process is of particular interest when the benzaldehyde is 3-phenoxybenzaldehyde and the acid halide is 2 - (4 - chlorophenyl) - 3 - methylbutanoic halide, 2,2,3,3-tetramethylcyclopropane-carboxylic halide or 2 - (2,2 - dichlorovinyl) - 3,3 - dimethylcyclopropanecarboxylic halide, because the esters formed therefrom, i.e., *alpha* - cyano - 3 - phenoxybenzyl 2-(4 - chlorophenyl) - 3 - methylbutanoate, *alpha* - cyano - 3 - phenoxybenzyl 2,2,3,3-tetramethylcyclopropanecarboxylate and *alpha*-cyano-3-phenoxybenzyl 2 - (2,2 - dichlorovinyl) - 3,3 - dimethylcyclopropanecarboxylate, respectively, are highly-active pesticidal compounds.

The ester of the general formula I may be recovered from the reaction mixture obtained by

isolating the organic phase from the aqueous phase, extracting the aqueous phase with a hydrocarbon solvent, combining the extract phase with the organic phase, washing the mixture thus obtained with water, drying the washed mixture and evaporating the solvent from the dried mixture. The aqueous raffinate phase contains an alkali metal salt of a carboxylic acid of the general formula RC(O)OH, R having the same meaning as in formula III. This carboxylic acid can be isolated by acdifying the aqueous phase and filtering off the resultant precipitated carboxylic acid.

The following Example further illustrates the invention. The reaction mixtures were analyzed by gas-liquid chromatography to determine the various yields. The solutions were dried over anhydrous sodium sulphate. Solvents were flashed off in a film evaporator at a pressure of 15 mm Hg.

Example

In the manufacture of *alpha*-cyano-3-phenoxybenzyl 2-(2,2-dichlorovinyl)-3,3-dimethylcyclopropylcarboxylate, the reactor was charged with 3-phenoxybenzaldehyde (1235 mol.), 2 - (2,2 - dichlorovinyl) - 3,3 - dimethylcyclopropanecarbonyl chloride (1409.5 mol) and xylene (550 kg). A solution of sodium cyanide (1530 mol.) and tetrabutylammonium bromide (0.12 mol) in water (305 kg) was then added gradually at 3°C over a period of 3.5 hours. After prolonged periods of stirring the 3-phenoxybenzaldehyde had not been completely converted and it was apparent that 2 - (2,2 - dichlorovinyl) - 3,3 - dimethylcyclopropanecarboxylic acid anhydride was present in the reaction mixture. Further quantities of acid chloride and tetrabutylammonium bromide were added to the reactor but this had not significant effect on the reaction mixture. Stirring was continued for 9 hours at 25°C until the composition of the reaction mixture became constant; the composition was as follows:

| | | |
|---|---|---|
| 2-(2,2-dichlorovinyl)-3,3-dimethylcyclopropane-carboxylic anhydride | 127 | mol. |
| 3-phenoxybenzaldehyde | 99 | mol. |
| sodium cyanide | 140 | mol. |
| tetrabutylammonium bromide | 5 | mol. |
| *alpha*-cyano-3-phenoxybenzyl 2-(2,2-dichlorovinyl)-3,3-dimethylcyclopropane carboxylate | 1156 | mol. |
| xylene | 550 | kg |
| water | 350 | kg |
| sodium chloride | 1410 | mol. |
| hydrogen cyanide | 254 | mol. |

An aqueous solution of sodium carbonate (240 mol. of $Na_2CO_3$ in 225 kg of water) was added to the reactor and stirring was continued for 30 minutes at 20°C. Then, stirring was stopped and the reaction mixture allowed to settle for one hour, resulting in a sharp separation of the aqueous phase from the organic phase. The 3-phenoxybenzaldehyde had been fully converted into *alpha*-cyano-3-phenoxybenzyl 2 - (2,2 - dichlorovinyl) - 3,3 - dimethylcyclopropane carboxylate.

## Claims

1. A process for the preparation of an ester of the general formula:—

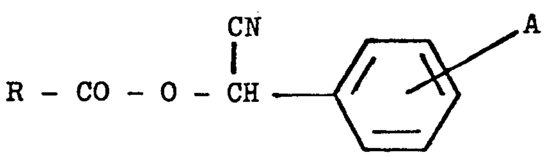

(I)

wherein R represents an optionally-substituted hydrocarbyl group and A represents a hydrogen atom or a phenoxy, phenylthio or benzyl group by reacting a benzaldehyde of the general formula:—

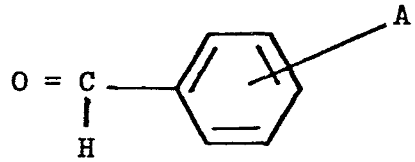

(III)

wherein A has the same meaning as in formula I with an acid halide of formula R—CO—Hal where Hal is chlorine or bromine and R has the same meaning as in formula I in the presence of water and a water-soluble cyanide and optionally in the presence of a phase transfer catalyst characterised in that when the reaction slows or comes to a standstill a compound capable of generating cyanide ions is added to the reaction mixture so that by-product acid anhydride of the general formula:—

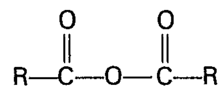

wherein R has the same meaning as in formula I is caused to react with the remaining aldehyde of formula II and with the generated cyanide ions to form further ester of general formula 1.

2. A process according to claim 1 characterised in that the compound capable of generating cyanide ions is an alkaline compound.

3. A process according to claim 2 characterised in that the alkaline compound is an alkali metal carbonate or hydroxide.

**Revendications**

1. Un procédé pour la préparation d'un ester de la formule générale:

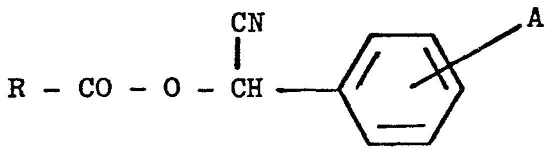

(I)

dans laquelle R représente un group hydrocarbyle éventuellement substitué et A représente un atome d'hydrogène ou un groupe phénoxy, phénylthio of benzyle, en faisant réagir un benzaldéhyde de la formule générale:

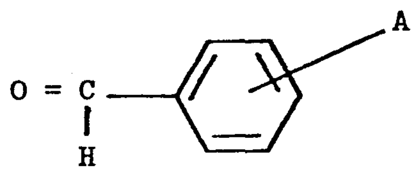

(II)

dans laquelle A a la même signification que dans la formule I avec un halogénure d'acide de formule R—CO—Hal dans laquelle Hal est du chlore ou du brome et R a la même signification que dans la formule I en présence d'eau et d'un cyanure soluble dans l'eau et éventuellement en présence d'un catalyseur de transfert de phase, caractérisé en ce que quand la réaction se ralentit ou s'arrête, on ajoute au mélange réactionnel un composé capable de produire des ions cyanure de manière que le sous-produit anhydride d'acide de la formule générale:

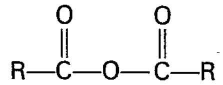

(III)

dans laquelle R a la même signification que dans la formule I réagisse avec l'aldéhyde de formule II restant et avec les ions cyanure produits pour former une quantité supplémentaire d'ester d formule générale I.

2. Un procédé selon la revendication 1, caractérisé en ce que le composé capable de produire des ions cyanure est un composé alcalin.

3. Un procédé selon la revendication 2, caractérisé en ce que le composé alcalin est un carbonate ou hydroxyde de métal alcalin.

**Patentansprüche**

1. Verfahren zur Herstellung eines Esters der allgemeinen Formel:

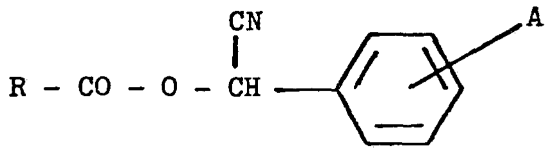

(I)

in der R eine gegebenenfalls substituierte Kohlenwasserstoffgruppe und A ein Wasserstoffatom oder eine Phenoxy-, Phenylthio- oder Benzylgruppe bedeuten, durch Umsetzen eines Benzaldehyds der allgemeinen Formel:

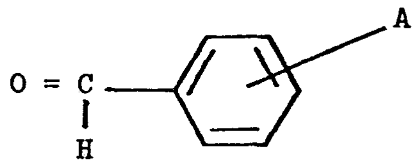

(II)

in der A die gleiche Bedeutung wie in Formel (I) hat, mit einem Säurehalogenid der Formel R—CO—Hal, in der Hal für Chlor oder Brom steht und R die gleiche Bedeutung wie in Formel (I) hat, in Gegenwart von Wasser und einem wasserlöslichen Cyanid und gegebenenfalls in Gegenwart eines Phasentransferkatalysators, dadurch gekennzeichnet, dass, wenn die Reaktion langsamer wird oder zum Stillstand kommt, eine Verbindung, die Cyanidionen erzeugen kann, zu dem Reaktionsgemisch gegeben wird, so dass das als Nebenprodukt entstandene Säureanhydride der allgemeinen Formel:

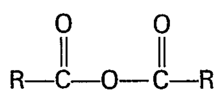

(III)

in der R die gleiche Bedeutung wie in Formel (I) hat, veranlasst wird, mit dem übrigen Aldehyd der Formel (II) und mit den erzeugten Cyanidionen unter Bildung von weiterem Ester der allgemeinen Formel (I) zu reagieren.

2. Verfahren nach Anspruch 1, dadurch gekennzeichnet, dass die Verbindung, die Cyanidionen erzeugen kann, eine alkalische Verbindung ist.

3. Verfahren nach Anspruch 2, dadurch gekennzeichnet, dass die alkalische Verbindung ein Alkalicarbonat oder ein Alkalihydroxid ist.